# EUROPÄISCHE PATENTSCHRIFT

(11) **EP 0 738 733 B1**
(45) Veröffentlichungstag und Bekanntmachung des Hinweises auf die Patenterteilung: **04.10.2001**
(21) Anmeldenummer: 96104923.6
(22) Anmeldetag: 28.03.1996
(51) Int. Cl.: C07H 1/06, B01D 15/08, B01L 3/00

(54) **Vorrichtung zur Isolierung von Nukleinsäuren**
Method for isolating nucleic acids
Méthode pour l'isolation des acides nucléiques

(30) Priorität: 01.04.1995 DE 19512369
(43) Veröffentlichungstag der Anmeldung: 23.10.1996
(73) Patentinhaber: Roche Diagnostics GmbH, 68298 Mannheim (DE)
(72) Erfinder: Walter, Thomas, Dr., 83673 Bichl (DE); Fritz, Michael, 68647 Bilbis (DE); Harttig, Herbert, Dr., 67122 Altrip (DE); Lange, Hans, 68623 Lampertheim (DE); Lerch, Rolf, 68549 Ilvesheim (DE)

(56) Entgegenhaltungen:
- EP-A- 0 673 679
- EP-A- 0 676 643
- EP-A- 0 687 502
- WO-A-91/07648
- DE-A- 4 127 276

## Beschreibung

Gegenstand der Erfindung ist eine Vorrichtung zur Isolierung von Nukleinsäuren, ein Verfahren zur Herstellung dieser Vorrichtung und ein Verfahren zur Isolierung von Nukleinsäuren mit Hilfe dieser Vorrichtung.

In jüngerer Zeit haben sich Nukleinsäuren als Analyten in chemischen Nachweisverfahren immer weiter durchgesetzt, da sie eine sehr spezifische Diagnose von Krankheiten erlauben. So ist beispielsweise der Nachweis von viralen oder bakteriellen Infektionen oder die Bestimmung einer genetischen Disposition für eine Krankheit durch die Analyse von Nukleinsäuresequenzen sehr differenziert möglich. Dies wurde unter anderem durch die Einführung der Amplifikationsverfahren von Nukleinsäuren ermöglicht. Dennoch sind die zur Verfügung stehenden Mengen an Nukleinsäuren in einer Probe recht gering im Vergleich zu weiteren Komponenten der Probenflüssigkeiten. Viele dieser Komponenten können auch die Bestimmung von Nukleinsäuren stören. Aus diesem Grund hat es sich als zweckmäßig erwiesen, die Nukleinsäuren vor der Durchführung des eigentlichen Nachweisverfahrens von dem Großteil der in einer Probe enthaltenen Komponenten abzutrennen.

Schon früh wurde hierfür die Adsorption von Nukleinsäuren an feste Phasen vorgeschlagen. So wurde beispielsweise in Proc. Natl. Acad. Sci. U.S.A. 76, 615-619 (1979) die Eigenschaft von Nukleinsäuren in Gegenwart chaotroper Salze an Glasoberflächen zu binden, beschrieben. Hier wurde gemahlenes Flintglas für die Isolierung von Nukleinsäurefragmenten aus Agarose Gelen in Kombination mit einer gesättigten Lösung von NaI zur Auflösung der Agarose-Matrix verwendet. Hierbei wurden mehr als 95 % der Nukleinsäuren nach 2 Stunden bei 25 °C gebunden, wobei ca. 1 µg DNA/mg Glaspartikel gebunden wurden. Anschließend wurden die Glaspartikel mehrmals gewaschen und anschließend die Nukleinsäuren eluiert. Es ergab sich für diese Folge von Schritten ein Zeitbedarf von mindestens 3 Stunden.

Zur Verbesserung der Handhabung und Verkürzung des Zeitbedarfs wurden alternative Methoden zur Reinigung von Nukleinsäuren an Glasoberflächen entwickelt. WO 91/07648 beschreibt ein zylindrisches Kunststoffgefäß, das am Boden eine Kunststoffmembran enthält, die auch Glasfasern enthalten kann. Auch hier sind mehrere Wasch- und Elutionsschritte erforderlich. Ein ähnliches Verfahren ist auch in DE-A-4127276 beschrieben, wobei die nukleinsäurehaltigen Lösungen nur kurze Zeit mit der Glasfaseroberfläche in Kontakt stehen. Entsprechende Produkte sind über die genannten Patentanmelder erhältlich. Im ersten Fall wird als Bindungskapazität 50 ng bis 30 µg in einer Größenverteilung von 100 bis 23.000 bp angegeben. Die Ausbeute wird mit 60 bis 85 % beziffert. Um die maximale Effizienz zu erreichen, wird vom Hersteller empfohlen, den Durchlauf der Probe mindestens auf zweimal aufzutragen, da sonst die Ausbeute um mindestens 25 % reduziert ist. Für Fragmente mit einer Größe von mehr als 23.000 bp beträgt die Ausbeute höchstens 40 %.

Die angebotenen Zentrifugationsröhrchen enthalten je nach erforderlicher Bindungskapazität mehrere Lagen Glasfaservlies in einem Gefäß mit stark konischem Boden. Mittig im Boden befindet sich eine kleine Auslauföffnung. Die Herstellung der Zentrifugationsröhrchen geschieht so, daß zunächst das Glasfaservlies von oben in das Gefäß eingeführt und auf den Boden gedrückt wird. Anschließend wird durch die gleiche Öffnung, durch die später die Nukleinsäurelösung aufgegeben wird, ein Ring in das Gefäß eingeführt, der durch Kontakt mit der Innenwand des Gefäßes das Glasfaservlies am Boden festdrückt und somit ein Aufschwimmen des Vlieses während der Inkubation mit der nukleinsäurehaltigen Probe verhindert. Diese Art der Konstruktion des Zentrifugationsröhrchen hat, wie nun festgestellt wurde, den Nachteil, daß viele störende Probenkomponenten in ihr zurückgehalten werden, so daß mehrere Waschschritte zur vollständigen Entfernung erforderlich werden. Dies wiederum führt zu einer verringerten Ausbeute an Nukleinsäuren oder zur Störung nachfolgender enzymatischer Reaktionen. Für ein auf dem Markt erhältliches Produkt wird eine Bindekapazität von 5 µg bei 90 % Ausbeute mit einem 100 bp langen Fragment angegeben. In einem weiteren Produkt wird eine Ausbeute von 75 bis 90 % bei einer Bindekapazität von 20 µg erreicht werden.

In dem bisher genannten Stand der Technik wird nur die Isolierung von DNA, nicht jedoch die Isolierung von Gesamt-Nukleinäsuren oder RNA beschrieben.

Gegenstand der Erfindung war daher die Verbesserung der Produkte zur Isolierung von Nukleinsäuren sowie deren Herstellung.

Diese Aufgabe wird gelöst durch eine Vorrichtung zur Isolierung von Nukleinsäuren aus einer flüssigen Probe mit einer Eintritts- und einer Austrittsöffnung und einem zwischen diesen Öffnungen gelegenen Material zur Bindung der Nukleinsäuren, wobei die Austrittsöffnung nach Einbringen des Materials verengt wurde. Das erfindungsgemäße Gefäß kann fast jede beliebige äußere Form aufweisen. Bevorzugt hat sie jedoch eine im wesentlichen zylindrische oder auch zumindest in Teilen konische Form. Eine besonders bevorzugte Ausführungsform dieses Gefäßes hat die Form einer Röhre, z. B. eines gängigen Zentrifugenröhrchens. Solche Zentrifugenröhrchen haben eine Länge von ca. 25 mm und einen Durchmesser von ca 8.5 mm. Bevorzugt weist diese Röhre einen der Eintrittsöffnung zugewandten Teil auf, der für die Aufnahme der nukleinsäurehaltigen Probe ausgestaltet ist. Insbesondere ist das Volumen dieses Raumes so groß, daß die Probe vollständig aufgenommen werden kann. An der Eintrittsöffnung können zusätzlich Bauelemente vorgesehen sein, die den Verschluß der Eintrittsöffnung durch einen Deckel ermöglichen. Solche Konstruktionen sind z. B. aus Eppendorf-Hütchen bekannt.

Das Material zur Bindung der Nukleinsäuren befindet sich bevorzugt in dem Bereich der Vorrichtung, der sich in der Nähe der Austrittsöffnung befindet. Dies bedeutet, daß der Raum, welcher sich in der Röhre zwischen dem Material und der Austrittsöffnung befindet, bevorzugt kleingehalten wird, um die Gefahr des Zurückhaltens von Flüssigkeitströpfchen uns somit die Kontaminationsgefahr gering zu halten. Das Material zur Bindung von Nukleinsäuren ist in der Vorrichtung fixiert. In Richtung auf die Eintrittsöffnung zu ist hierzu eine nicht ohne Zerstörung der Vorrichtung zu entfernende Haltevorrichtung vorgesehen. Sie ist bevorzugt aus dem selben Material wie das Gefäß. In dieser Ausführungsform ist die Vorrichtung besonders vorteilhaft, da dann das erfindungsgemäße Gefäß inklusive Haltevorrichtung im Spritzgußverfahren herstellbar ist. Bevorzugt ragt die Haltevorrichtung von der Innenwand des Gefäßes in den Innenraum hinein und ist in Richtung auf die Eintrittsöffnung abgeschrägt. Diese Anforderungen können beispielsweise durch einen umlaufenden Rand bzw. Vorsprung in den Innenraum gelöst werden. Die Abschrägung in Richtung auf die Eintrittsöffnung gewährleistet, daß sich in dem Innenraum nur stumpfe Winkel ergeben. Als ein wesentlicher Nachteil der bekannten Gefäße hat sich nämlich erwiesen, daß in rechten oder spitzeren Winkeln und im Fall, daß ein Ring nicht perfekt an der Innenwand des Gefäßes anliegt, Flüssigkeiten, z. B. die Probenflüssigkeit, zurückgehalten werden, und somit die Effizienz der Waschschritte verringert ist. Darüber hinaus wird bei der Herstellung dieses Gefäßes ein relativ kritischer Verfahrensschritt, nämlich das Einsetzen des Ringes, eingespart. Durch die Vorsehung der Haltevorrichtung schon bei der Herstellung des Gefäßes ist man darüber hinaus bei der Wahl der Haltevorrichtungen flexibler und kann daher das Aufeinandertreffen von Bauteilen in spitzen Winkeln vermeiden.

Die Haltevorrichtungen können jedoch auch nicht umlaufende Bauteile sein, z. B. regelmäßig oder unregelmäßig auf vergleichbarer Höhe angeordnete Vorsprünge. Materialien zur Bindung von Nukleinsäuren sind bekannt. Besonders bevorzugt als Material sind Materialien mit einer Oberfläche aus Glas. Dieses Material wird besonders bevorzugt in Form eines Vlieses eingesetzt, da Vliese sehr einfach in die Form der Innenwand der erfindungsgemäßen Vorrichtung eingepaßt werden können, ohne daß sich während der Benutzung des Materials zur Isolierung von Nukleinsäuren eine wesentliche Änderung dieser äußeren Form ergibt. Außerdem sind Vliese leicht durchströmbar für Flüssigkeiten. Als besonders bevorzugt haben sich Vliese mit einer Dichte von zwischen 40 und 100 mg/ccm(g/cdm) erwiesen.

In einer bevorzugten Ausführungsform ist das nukleinsäurebindende Material gegen die Austrittsöffnung hin vollflächig durch eine flüssigkeitsdurchlässige poröse Matrix abgestützt. Diese Matrix wird im folgenden auch als Stützvlies bezeichnet. Eine solche flüssigkeitsdurchlässige Matrix sind beispielsweise Vliese aus Materialien, die gegenüber den Komponenten der Flüssigkeit relativ inert sind und möglichst wenig Komponenten binden oder solche, die Nukleinsäure binden. Als besonders bevorzugt hat sich hierbei Polyester oder Polypropylen erwiesen. Diese Matrix trennt das nukleinsäurebindende Material bevorzugt praktisch vollständig (d. h. zu mehr als 90 %) gegen die Austrittsöffnung ab, so daß keine Flüssigkeit von dem nukleinsäurebindenden Material direkt in die Austrittsöffnung fließen kann. Diese Matrix hat zweierlei Funktion. Zunächst dient sie zum Zurückhalten eventuell nicht fest verankerter Fasern des nukleinsäurebindenden Materials (Ausbeuteverbesserung). Darüber hinaus gibt diese Matrix die Möglichkeit, die Austrittsöffnung des Gefäßes zunächst so breit zu halten, daß das nukleinsäurebindende Material durch sie in das Gefäß eingeführt werden kann und anschließend die Austrittsöffnung verengt werden kann. Für den Fall, daß das nukleinsäurebindende Material aus einer partikulären Matrix besteht, ist dieses Material auch in Richtung auf die Eintrittsöffnung durch eine flüssigkeitsdurchlässige poröse Matrix abgestützt, um eine Aufwirbeln des Materials in den Probenaufnahmeraum zu vermeiden.

Die erfindungsgemäße Vorrichtung ist dadurch charakterisiert, daß die Austrittsöffnung nach Einbringen des nukleinsäurebindenden Materials verengt wurde. Die Austrittsöffnung ist bevorzugt so weit verengt, daß das nukleinsäurebindende Material bzw. die stützende Matrix nicht aus der Austrittsöffnung entweichen kann. Je fester das Material bzw. die Matrix ist, desto weniger stark muß die Verengung sein. Bevorzugt ist die Verengung ein Rand, welcher sich von der Röhrchenwand umlaufend nach innen erstreckt, so daß beispielsweise bei einem Röhrchendurchmesser von 8 mm der Rand mindestens 0.5 mm, bevorzugt zwischen 0.75 und 1.5 mm von jeder Seite in den Röhrcheninnenraum hereinragt. Die untere Grenze des Hineinragens wird durch das Festhalten des Materials bzw. der Matrix limitiert, während die obere Grenze durch das möglichst vollständige Aussaugen des Vlieses durch die Austrittsöffnung limitiert wird.

Ebenfalls Gegenstand der Erfindung ist ein Verfahren zur Herstellung eines Gefäßes zur Isolierung von Nukleinsäuren aus einer flüssigen Probe mit einer Eintritts- und einer Austrittsöffnung und einem zwischen diesen Öffnungen gelegenen Material zur Bindung der Nukleinsäuren durch Einführen des Materials durch die Austrittsöffnung in Richtung auf die Eintrittsöffnung bis zu einer im Gefäß befestigten Haltevorrichtung und Verengung der Austrittsöffnung, so daß das Material zwischen Haltevorrichtung und der Verengungsstelle festgehalten wird. Die Verengung kann beispielsweise geschehen durch Umbördeln, z. B. thermisch, oder durch thermische Fixierung. Dieses Herstellverfahren ist besonders einfach und im Hinblick auf die Fixierung des Materials besonders sicher.

Das erfindungsgemäße Gefäß ist bevorzugt aus einem spritzgußfähigen Material hergestellt, z. B. Polyethylen, Polypropylen, Polycarbonat oder Polyurethan, besonders bevorzugt Polypropylen.

Ebenfalls Gegenstand der Erfindung ist die Verwendung des erfindungsgemäßen Materials zur Isolierung von Nukleinsäuren, insbesondere ein Verfahren zur Isolierung von Nukleinsäuren aus einer flüssigen Probe durch Einbringen der Probe in das erfindungsgemäße Gefäß durch eine Eintrittsöffnung, Durchtritt der Probe durch das Material und Aufhebung der Bindung der Nukleinsäuren an das Material.

In einem typischen Verfahren wird die Probe durch Pipettieren einer gewünschten Menge an Probenflüssigkeit in das Gefäß eingebracht. Danach wird der Durchtritt der Probe durch das Material veranlaßt. Dies kann beispielsweise geschehen durch Zentrifugation, wobei die Flüssigkeit durch das Material und die Austrittsöffnung aus dem Gefäß herausgeschleudert wird. Dabei werden die Nukleinsäuren an der Matrix immobilisiert, während die restliche Probenflüssigkeit austritt. Bevorzugt wird die Matrix anschließend einmal mit einem Waschpuffer mittleren Salzgehaltes gewaschen, wodurch Reste der Probenflüssigkeit und nicht zu isolierende Komponenten aus dem Material entfernt werden.

Danach können die an der Matrix immobilisierten Nukleinsäuren durch einen Niedrigsalzpuffer von der Matrix eluiert werden. Solche Puffer sind aus DE 3724442 und Analytical Biochem. 175, 196 - 201 (1988) bekannt. Bevorzugt wird ein Puffer mit niedrigem Salzgehalt, kleiner 200 mM NaCl verwendet, z. B. 10 mM Tris-HCl, 1 mM EDTA, pH 8.0 oder H₂O. Für die erfindungsgemäße Matrix ergeben sich relativ hohe Ausbeuten.

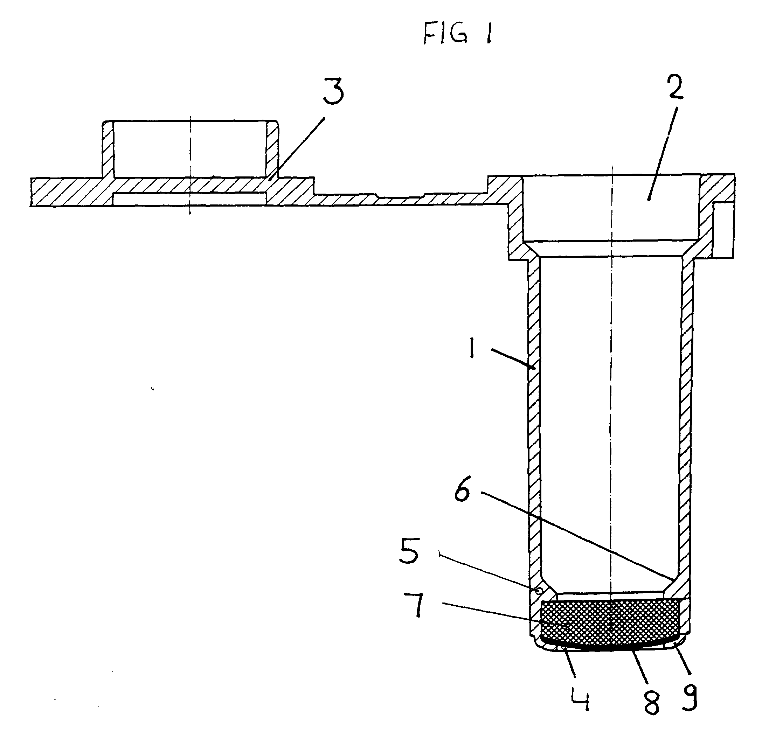

In Fig. 1 ist ein besonders geeignetes erfindungsgemäßes Gefäß im Querschnitt gezeigt.

Das gezeigte erfindungsgemäße Gefäß besteht aus einem im wesentlichen zylindrischen Teil 1, der an einem Ende eine Eintrittsöffnung 2 aufweist, die mit einem Deckel 3 verschlossen werden kann. In Richtung auf die Austrittsöffnung 4 findet man die Haltevorrichtungen 5, deren Abschrägungen 6 klar erkenntlich sind. Die Haltevorrichtungen sind in der bevorzugten Ausführungsform ein umlaufender Rand aus dem selben Material wie der zylindrische Körper 1. An die Haltevorrichtungen schließt sich das nukleinsäurebindende Material 7 an. Gegenüber der Austrittsöffnung 4 ist das Material durch ein Stützvlies 8 abgegrenzt. Das Stützvlies wird durch den Rand 9 innerhalb des Gefäßes festgehalten.

In Fig. 2 ist ein Röhrchen vor Bestückung mit dem nukleinsäurebindenden Material gezeigt.

In Fig. 3A und 3B ist die Bestückung des Röhrchens mit dem nukleinsäurebindenen Material 7 und einem Stützvlies 8 gezeigt.

In Fig. 4 ist das fertige erfindungsgemäße Röhrchen gezeigt.

In Fig. 5 ist ein Röhrchen gezeigt, dessen Bördelkante Schlitze aufweist, (Längsschnitt, Maße in mm).

In Fig. 6 ist das Röhrchen von Fig. 5 von unten gezeigt.

In Fig. 7 ist das Röhrchen von Fig. 5 analog Fig. 3A im Zustand vor Umbördelung des Rands gezeigt. Die Schlitze sind gestrichelt gezeichnet.

Die Erfindung wird durch die folgenden Beispiele weiter erläutert:

### Beispiel 1:

### Herstellung eines erfindungsgemäßen Zentrifugationsröhrchen

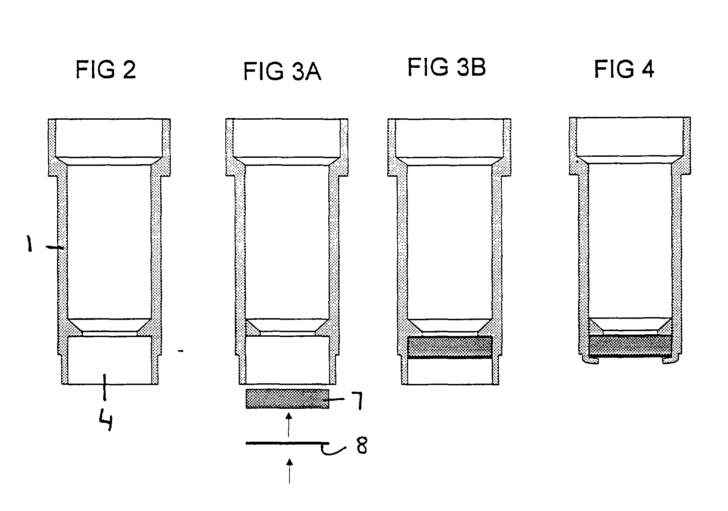

Im Spritzgußverfahren wird der Grundkörper 1 eines in Figur 2 im Längsschnitt dargestellten Zentrifugationsröhrchens, welches eine Länge von ca. 25 mm und einen Durchmesser von ca. 8.5 mm hat, aus Polypropylen PPN 1060 natur hergestellt. In der Spritzgußmaschine werden je ein Glasfaservlies (WF 264 Whatman, Flächengewicht 60 g/m²) 7 und ein Stützvlies 8 zugeführt und ausgestanzt. Je nach gewünschter Bindekapazität werden mehrere Glasvliese 7 eingesetzt, z. B. 7 Stück. Die ausgestanzten Vliese werden in das Zentrifugationsröhrchen wie in Figur 3A und 3B dargestellt von der Seite der Austrittsöffnung eingebracht. Danach wird die Austrittsöffnung 4 durch Umformen des noch etwa 60 °C warmen Kunststoff mit einem Stempel soweit verengt, daß die beiden Vliese wie in Figur 4 dargestellt fest fixiert sind.

### Beispiel 2:

### Isolierung von Plasmid DNA aus E.coli Kulturen

Anzucht von E. coli x pUC19: 25 ml LB-Medium + Ampicillin (150 µg/ml) wurden mit einer Einzelkolonie angeimpft und über Nacht bei 37°C unter schütteln bebrütet. Die OD₆₀₀-Messung ergab einen Wert von 2,0 (entspricht ca. 2 x 10⁹ Zellen/ml). Es wurden verschiedene E. coli Stämme verwendet.

Isolierung der Plasmid DNA: 1.5 ml der Bakterienkultur werden in ein Eppendorf-Reaktionsgefäß überführt und für ca. 30 Sekunden bei maximaler Geschwindigkeit in einer Eppendorf Tischzentrifuge Typ 5415C zentrifugiert. Der Überstand wird abgenommen, das Sediment wird in 250 µl Puffer (50 mM Tris-HCl, 10 mM EDTA, 100 mg/ml RNase A, pH 8,0) vollständig resuspendiert und vorsichtig mit 250 µl Puffer II (0,2 M NaOH, 1 % Natrium-dodecylsulfat) versetzt. Die Zellen lysieren vollständig und ergeben eine klare Lösung. Der Ansatz wird mit 350 µl Puffer III (2,9 M Guanidinium-HCl, 0,65 M K-Acetat, pH 4,15) gemischt und für ca. 3 min auf Eis inkubiert. Es bildet sich ein weißes Präzipitat, daß durch Zentrifugation sedimentiert wird. ein Filtertube wird in ein Auffanggefäße gesteckt und der Überstand wird auf das Filtertube aufgetragen. Durch kurze Zentrifugation wird die Flüssigkeit durch das Glasvlies transferiert. Der Durchlauf wird verworfen und es werden 500 µl Waschpuffer (5 M Guanidinium-HCl, 20 mM Tris, 37 % Ethanol, pH 6,65) aufgetragen und zentrifugiert. Die Verwendung von Waschpuffer I ist optional für besonders nuclease-reiche E. coli Stämme. Anschließend werden 500 µl Waschpuffer II (20 mM NaCl, 2 mM Tris, 80 % Ethanol, pH 7,5) auf das Filtertube aufgetragen und wie vor zentrifugiert. Das Filtertube wird nun in ein neues Eppendorf Reaktionsgefäß überführt und die Plasmid DNA in 100 ml TE (10 mM Tris-HCl, 0,1 mM EDTA, pH 8,5) eluiert.

Tabelle 1 gibt die Ausbeute und Reinheit der Isolierten Plasmid DNA wieder:

**Tabelle 1:**

| Ausbeute und Reinheit von isolierter Plasmid DNA aus verschiedenen E. coli Stämmen. | | | | |
|---|---|---|---|---|
| **Stamm** | **Waschpuffer I** | **Waschpuffer II** | **Ausbeute [µg]** | **OD**_{**260**}**/OD**_{**280**} |
| XL 1 blue | ja | ja | 8,9 | 1,8 |
| XL 1 blue | nein | ja | 9,4 | 1,82 |
| DH5α | ja | ja | 4,2 | 1,52 |
| DH5α | nein | ja | 4,2 | 1,62 |
| HB101 | ja | ja | 4,7 | 1,7 |
| HB101 | nein | ja | 5,8 | 1,77 |

### Beispiel 3:

### Reinigung von Reaktionsprodukten der Polymerase Ketten Reaktion (PCR)

Abtrennverhalten des Filtertubes: 10 µg Molekulargewichtsstandard VIII (Boehringer Mannheim, Fragmentlängen [bp]: 1114, 900, 692, 501, 489, 404, 320, 242, 190, 147, 124, 110, 67, 37, 34, 26, 19) in 100 µl TE, werden mit Rinderserumalbumin (20 mg/ml) versetzt. zu dieser Mischung werden 500 µl NA-Bindungspuffer (3 M Guanidinium-Thiocyanat, 10 mM Tris-HCl, 5 % Ethanol (v/v), pH 6,6, 25°C) gegeben und sorgfältig gemischt. Die Lösung wird auf ein Filtertube aufgetragen und ca. 30 Sekunden in einer EppendorfTischzentrifuge Typ 5415C zentrifugiert. Der Durchlauf wird Aufgefangen, er kann für spätere Analysen aufbewahrt werden. Das Vlies wird mit 500 µl Waschpuffer II gewaschen anschließend durch kurze Zentrifugation getrocknet. Die gebundene Nucleinsäure wird mit 50 µl TE eluiert.

Gelelektrophorese und Silberfärbung: Zur Überprüfung der Abtrennung des Modellproteins RSA und kleiner DNA Fragmente wird eine Polyacrylamid Gelelektrophorese mit dem Phastsystem (Pharmacia) durchgeführt.

4 µl des Eluates wird mit 1 µl 5 x Proben-Puffer versetzt. Als Kontrolle werden 4 µl Längenstandard VIII sowie 4 µl einer 1 : 100 Verdünnung der RSA Lösung in TE-Puffer ebenfalls mit 1 µl 5 x Proben-Puffer versetzt.

Die Proben werden kurz in der Eppendorf-Zentrifuge anzentrifugiert und 3 µl der Proben werden auf einen 6/4 Kamm (Pharmacia) aufgetragen und in einem Phast Gel, Gradient 8 - 25 % (Pharmacia) unter Verwendung von Native Buffer Strips (Pharmacia) aufgetrennt.

Anschließend werden die Proben in der Phast System Development Unit (Pharmacia) durch Silberfärbung detektiert.

Auswertung: Die einzelnen DNA Fragmente aus dem Eluat sind entsprechend der Kontrolle Längenstandard VIII aufgetrennt. Die Fragmente < 100 bp im Eluat sind durch die Reinigung mit dem Filtertube abgetrennt worden. Außerdem ist keine Proteinbande entsprechend der RSA Kontrollspur sichtbar.

| Phast Gel Separation Programm: | | | | | | |
|---|---|---|---|---|---|---|
| 1) | Vorlauf | 400 V | 10 mA | 2,5 W | 15°C | 100 Vh |
| 2) | Auftrag | 400 V | 1 mA | 2,5 W | 15°C | 5 Vh |
| 3) | Trennung | 400 V | 10 mA | 2,5 W | 15°C | 45 Vh |

Gele nach Auftrennung für 5 Minuten in 25 % Trichloressigsäure-Lösung inkubieren.

| Phast Gel Development Programm: | | | |
|---|---|---|---|
| 1) | Glutaraldehyd, 5 % | 5 Minuten | 50°C |
| 2) | H₂O | 2 Minuten | 50°C |
| 3) | H₂O | 2 Minuten | 50°C |
| 4) | AgNO₃, 0,4 % | 10 Minuten | 40°C |
| 5) | H₂O | 0,5 Minuten | 30°C |
| 6) | H₂O | 2,5 Minuten | 30°C |
| 7) | NaHCO₃ / Formaldehyd | 0,5 Minuten | 30°C |
| 8) | NaHCO₃ / Formaldehyd | 4 Minuten | 30°C |
| 9) | Ethanol, 10 % /Essigsäure, 5 % | 2 Minuten | 50°C |
| 10) | Glycerin, 5 % /Essigsäure, 10 % | 3 Minuten | 50°C |

Reinigung von PCR Reaktionsprodukten: DNA des Phagen Lambda wird als Matrize für die Herstellung von PCR Produkten folgender Länge verwendet: 500 bp, 750 bp, 1500 bp 3000 bp. Folgende PCR Ansätze werden zusammen pipettiert, gemischt und in einem Thermocycler Perkin-Elmer Typ 9600 inkubiert.

| Länge PCR Produkt [bp] | 500 | 750 | 1500 | 3000 |
|---|---|---|---|---|
| H₂O | 372,5 µl | 372,5 µl | 372,5 µl | 372,5 µl |
| dNTP [10 mM] | 40 µl | 40 µl | 40 µl | 40 µl |
| λ-DNA [1 ng/µl] | 25 µl | 25 µl | 25 µl | 25 µl |
| 10 x Reaktionspuffer | 50 µl | 50 µl | 50 µl | 50 µl |
| Primer 1 | 5 µl | 5 µl | 5 µl | 5 µl |
| Primer 2 | 5 µl | | | |
| Primer 3 | | 5 µl | | |
| Primer 5 | | | 5 µl | |
| Primer 7 | | | | 5 µl |
| Primer 10 | | | | |
| Taq-Polymerase [U] | 2,5 | 2,5 | 2,5 | 2,5 |

Alle Reagenzien aus Boehringer Mannheim PCR Core Kit (Kat. No. 1 578 553)

| Thermocycler-Programm für 500, 750 und 1500 bp: | | 3000 bp: | |
|---|---|---|---|
| 2 Min | 94°C | 2 Min | 94°C |
| 10 Cyclen: | | 10 Cyclen: | |
| 10 Sek. | 94°C | 10 Sek. | 94°C |
| 30 Sek. | 55°C | 30 Sek. | 55°C |
| 1 Min | 72°C | 5 Min | 72°C |
| 20 Cyclen: | | 20 Cyclen: | |
| 10 Sek. | 94°C | 10 Sek. | 94°C |
| 30 Sek. | 55°C | 30 Sek. | 55°C |
| 1 Min | 72°C + 20 Sek./Zyklus | 5 Min | 72°C + 20 Sek./Zyklus |
| | | | |
| 7 Min | 72°C | 7 Min | 72°C |

Die PCR Produkte wurden wie oben Beschrieben gereinigt und im Phastgelsystem analysiert. Parallel wurde jeweils ein Aliquot des Reaktionsproduktes vor der Reinigung analysiert.

Auswertung: Im Vergleich vor der Reinigung sind bei allen PCR Produkten überschüssige Primer abgetrennt worden. Es konnten zwischen 1,4 und 4 µg PCR Produkt isoliert werden.

### Klonierung von PCR Produkten:

pUCIQ17 (3632 bp, Boehringer Mannheim) wurde mit dem Restriktionsenzym *Dra*II linearisiert und in folgendem PCR Ansatz amplifiziert:

| PCR Ansatz: | | PCR Programm (PE 9600): | |
|---|---|---|---|
| pUCIQ17 | 10 ng | 18 Cyclen: | |
| Primer 1 | 2 µM | 10 Sek. | 94°C |
| Primer 2 | 2 µM | 30 Sek. | 57°C |
| 10 x Puffer | 50 µl | 4 Min | 72°C |
| dNTP | 0,2 mM | | |
| Taq Polymerase | 2,5 U | | |
| H₂O | ad 500 µl | | |

Alle Reagenzien aus Boehringer Mannheim PCR Core Kit.

Die Primer binden bei Nukleotid 3500 und 3632 und ergeben ein 3493 bp PCR Produkt. Sie enthalten eine Schnittstelle für das Restriktionsenzym *Cla*I, diese Schnittstelle kommt im Plasmid pUKIQ17 nicht vor.

Im Anschluß an die PCR Reaktion wurde das Reaktionsprodukt nach der oben beschriebenen Methode gereinigt. Zum Vergleich wurde eine Reinigung durch Präzipitation mit Polyethylenglycol-Fällung (PEG) durchgeführt (Barnes W.M. (1992) *Gene* 112, 29). hierdurch werden störende Komponenten für die nachfolgende Religations-Reaktion entfernt.
Beide Proben werden mit dem Restriktionsenzym ClaI gespalten und über ein Agarosegel aufgetrennt. Die DNA Fragmente werden aus dem Agarosegel eluiert. Die hier verwendeten Methoden sind Standardverfahren und etwa beschrieben in Sambrook, J., Fritsch, E.F. & Maniatis, T. (1989) Molecular Cloning: A Laboratory Manual, 2nd edition, CSH Laboratory Press, Cold Spring Harbor, New York.

Die Proben (max. 30 ng) werden unter Verwendung des Rapid DNA Ligation Kit (Boehringer Mannheim, Kat. Nr. 1 635 379) nach Kit Vorschrift religiert und in kompetente E. coli DH5α Zellen transformiert. die Zellen werden auf TN Ap 100 X-Gal Platten ausgestrichen und über Nacht bei 37°C inkubiert. Am nächsten Tag werden die Kolonien ausgezählt, das Ergebnis ist in Tabelle 2 dargestellt.

| Reinigung | Filtertube | PEG |
|---|---|---|
| Zahl der Kolonien | 2212 | 2832 |

Die Anzahl der Kolonien ist bei Reinigung über Filtertube in der gleichen Größenordnung wie bei Reinigung über PEG. Die wesentlich einfachere Filtertube Methode liefert daher eine ähnlich saubere Nukleinsäure wie die wesentlich zeitaufwendigere PEG Methode.

### Beispiel 4

### Ausführungsform mit geschlitztem Bördelrand und kleinerer Austrittsöffnung

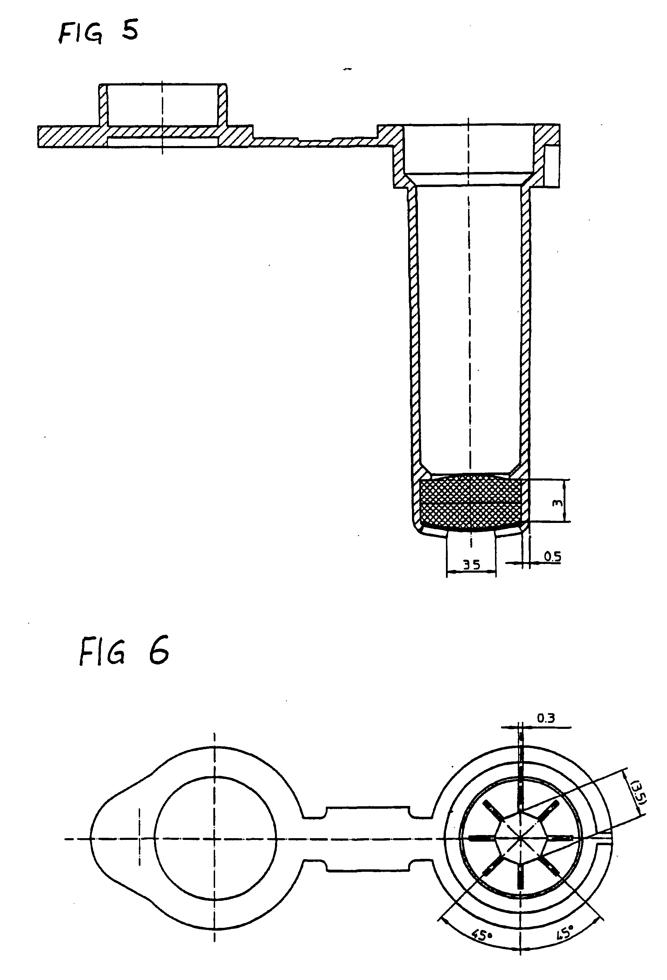

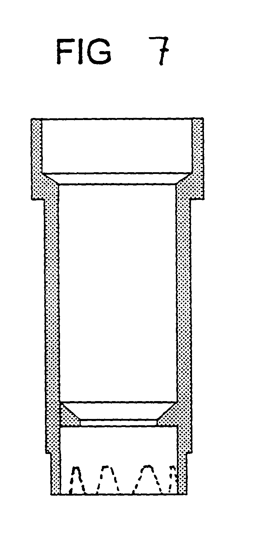

In einer weiteren Ausführungsform des erfindungsgemäßen Zentrifugationsröhrchens ist die Austrittsöffnung wie in Figur 5 dargestellt stärker verengt. In dieser Ausführungsform ist der Bördelrand der Auslaßöffnung (wie in Figur 6 dargestellt) geschlitzt. Mit dieser Anordnung ist es möglich, besonders hochviskose Lösungen zu zentrifugieren, ohne daß eine größere Menge an Restflüssigkeit im Device nach der Zentrifugation zurückbleibt.

### Beispiel 5

Durch die gewählte Ausführungsform des erfindungsgemäßen Zentrifugationsdevice in Beispiel 1 und Beispiel 4, wird vermieden, daß nach der Zentrifugation Flüssigkeit in toten Winkeln des Device zurückbleibt. Nicht vollständige Zentrifugation führt zu Ausbeuteverlusten, und zu geringerer Reinheit der isolierten Nukleinsäure.

| **Restflüssigkeit nach Einfüllen von 500 µl Wasser und anschließende Zentrifugation für 2 Minuten bei 6000 x g** | | | | | | |
|---|---|---|---|---|---|---|
| | | | | | | **MW.** |
| Zentrifugationsröhrchen gem. Beispiel 1 | 1,7mg | 1,6mg | 1,6mg | 1,4mg | 1,8mg | **1,6mg** |
| QIA Quick Device | 9,5mg | 9,9mg | 9,9mg | 10,2mg | 10,5mg | **10,0mg** |
| Zentrifugationsröhrchen gem. Beispiel 4 | 1,7mg | 2,0mg | 1,5mg | 2,3mg | 2,1mg | **1,9mg** |
| MW: Mittelwert | | | | | | |

Es wird klar, daß die erfindungsgemäßen Röhrchen erheblich weniger Restflüssigkeit zurückhalten. Dies ist wohl insbesondere auf die Abschrägung an der Haltevorrichtung zurückzuführen.

## Patentansprüche

1. Vorrichtung zur Isolierung von Nukleinsäuren aus einer flüssigen Probe mit einer Eintritts- und einer Austrittsöffnung und einem zwischen diesen Öffnungen gelegenen Material zur Bindung der Nukleinsäuren, **dadurch gekennzeichnet, daß** die Austrittsöffnung nach Einbringen des Materials verengt wurde.

2. Vorrichtung gemäß Anspruch 1, **dadurch gekennzeichnet, daß** das Material in Richtung der Eintrittsöffnung durch eine nicht ohne Zerstörung der Vorrichtung zu entfernende Haltevorrichtung festgehalten ist.

3. Vorrichtung gemäß Anspruch 2, **dadurch gekennzeichnet, daß** die Haltevorrichtung einstückig aus dem selben Material wie die Vorrichtung besteht.

4. Vorrichtung gemäß Anspruch 3, **dadurch gekennzeichnet, daß** die Haltevorrichtung von Innenwand des Gefäßes in den Innenraum hineinragt und in Richtung auf die Eintrittsöffnung abgeschrägt ist.

5. Vorrichtung gemäß Anspruch 4, **dadurch gekennzeichnet, daß** das Material gegen die Austrittsöffnung hin vollflächig durch eine flüssigkeitsdurchlässige poröse Matrix abgestützt ist.

6. Verfahren zur Herstellung einer Vorrichtung zur Isolierung von Nukleinsäuren aus einer flüssigen Probe mit einer Eintritts- und einer Austrittsöffnung und einem zwischen diesen Öffnungen gelegenen Material zur Bindung der Nukleinsäuren, **dadurch gekennzeichnet, daß** es folgende Schritte enthält:
- Einführen des Materials durch die Austrittsöffnung in Richtung auf die Eintrittsöffnung bis zu einer im Innenraum der Vorrichtung befestigten Haltevorrichtung und
- Verengung der Austrittsöffnung, so **daß** das Material zwischen Haltevorrichtung und der Verengungsstelle festgehalten wird.

7. Verfahren gemäß Anspruch 6, **dadurch gekennzeichnet, daß** die Verengung der Austrittsöffnung durch Umbördeln oder thermische Fixierung vorgenommen wird.

8. Verfahren zur Isolierung von Nukleinsäuren aus einer flüssigen Probe durch
- Einbringen der Probe in eine Vorrichtung gemäß Anspruch 1 durch die Eintrittsöffnung,
- Durchtritt der Probe durch das Material und
- Aufhebung der Bindung der Nukleinsäuren an das Material.

## Claims

1. Device for the isolation of nucleic acids for a liquid sample with an inlet and an outlet opening and a material for binding nucleic acids which is located between these openings, wherein the outlet opening is narrowed after introduction of the material.

2. Device as claimed in claim 1, wherein the material is held in the direction of the inlet opening by a holding device that cannot be removed without destroying the device.

3. Device as claimed in claim 2, wherein the holding device is manufactured in one piece from the same material as the device.

4. Device as claimed in claim 3, wherein the holding device extends from the inner wall of the vessel into the interior of the vessel and is bevelled towards the inlet opening.

5. Device as claimed in claim 4, wherein the material is supported over its whole area against the outlet opening by a liquid-permeable porous matrix.

6. Process for the manufacture of a device for the isolation of nucleic acids from a liquid sample having an inlet and outlet opening and a material for binding nucleic acids which is located between these openings, wherein it comprises the following steps:
- inserting the material through the outlet opening towards the inlet opening up to a holding device which is fixed in the interior of the device and
- narrowing the outlet opening so that the material is held between the holding device and the site of constriction.

7. Process as claimed in claim 6, wherein the narrowing of the outlet opening is achieved by flanging or by thermal fixation.

8. Process for the isolation of nucleic acids from a liquid sample by
- introducing the sample into a device as claimed in claim 1 through the inlet opening
- passage of the sample through the material and
- abolishing the binding of the nucleic acids to the material.

## Revendications

1. Dispositif pour l'isolation d'acides nucléiques provenant d'un échantillon liquide, avec une ouverture d'entrée et une ouverture de sortie et un matériau, disposé entre ces ouvertures, destiné à se lier aux acides nucléiques, **caractérisé en ce que** l'ouverture de sortie est rétrécie après l'apport du matériau.

2. Dispositif selon la revendication 1, **caractérisé en ce que** le matériau est immobilisé en direction de l'ouverture d'entrée par un dispositif de maintien qui ne peut être enlevé sans détruire le dispositif.

3. Dispositif selon la revendication 2, **caractérisé en ce que** le dispositif de maintien est réalisé dans le même matériau et d'un seul tenant avec le dispositif.

4. Dispositif selon la revendication 3, **caractérisé en ce que** le dispositif de maintien déborde de la paroi intérieure du récipient dans l'espace intérieur et est chanfreiné en direction de l'ouverture d'entrée.

5. Dispositif selon la revendication 4, **caractérisé en ce que** vers l'ouverture de sortie, le matériau est soutenu sur toute sa surface par une matrice poreuse perméable aux liquides.

6. Procédé de fabrication d'un dispositif pour l'isolation d'acides nucléiques provenant d'un échantillon liquide, avec une ouverture d'entrée et une ouverture de sortie et un matériau, disposé entre ces ouvertures, destiné à se lier aux acides nucléiques, **caractérisé en ce qu'**il comprend les étapes suivantes :
l'insertion du matériau par l'ouverture de sortie, en direction de l'ouverture d'entrée, jusqu'à un dispositif de maintien fixé dans l'espace intérieur du dispositif, et
le rétrécissement de l'ouverture de sortie de telle sorte que le matériau soit immobilisé entre le dispositif de maintien et l'emplacement du rétrécissement.

7. Dispositif selon la revendication 6, **caractérisé en ce que** le rétrécissement de l'ouverture de sortie est réalisé par rabattement ou fixation thermique.

8. Procédé d'isolation d'acides nucléiques provenant d'un échantillon liquide, par :
placement de l'échantillon dans un dispositif selon la revendication 1, à travers l'ouverture d'entrée,
traversée du matériau par l'échantillon, et
suppression de la liaison des acides nucléiques au matériau.


### Sequenzprotokoll

### SEQUENZPROTOKOLL

### (1) ALLGEMEINE ANGABEN:

(i) ANMELDER:
   (A) NAME: Boehringer Mannheim GmbH
   (B) STRASSE: Sandhoferstr. 116
   (C) ORT: Mannheim
   (E) LAND: DE
   (F) POSTLEITZAHL: 68298
   (G) TELEFON: 0621 759 4348
   (H) TELEFAX: 0621 759 4457
(ii) BEZEICHNUNG DER ERFINDUNG: Vorrichtung zur Isolierung von Nukleinsäuren
(iii) ANZAHL DER SEQUENZEN: 2
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)

### (2) ANGABEN ZU SEQ ID NO: 1:

(i) SEQUENZKENNZEICHEN:
   (A) LÄNGE: 34 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligodeoxyribonukleotid"
(iii) HYPOTHETISCH: NEIN
   (xi) SEQUENZBESCHREIBUNG: SEQ ID NO: 1:
   AGCTTATCGA TGGCACTTTT CGGGGAAATG TGCG 34

### (2) ANGABEN ZU SEQ ID NO: 2:

(i) SEQUENZKENNZEICHEN:
   (A) LÄNGE: 36 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligdeoxyribonukleotid"
(iii) HYPOTHETISCH: NEIN
(xi) SEQUENZBESCHREIBUNG: SEQ ID NO: 2:
   AGCTTATCGA TAAGCGGATG CCGGGAGCAG ACAAGC 36